# EUROPEAN PATENT APPLICATION

(11) **EP 2 305 187 A2**
(43) Date of publication of application: **06.04.2011**
(21) Application number: 10192837.2
(22) Date of filing: 17.06.2008
(51) Int. Cl.: A61F 5/443, A61F 5/445, A61L 15/58, A61L 28/00, A61L 24/00

(54) **A collecting device for body fluids**

(30) Priority: 25.04.2008 DK 200800589
(62) Divisional of application: 08758286.2
(71) Applicant: Coloplast A/S, 3050 Humlebaek (DK)
(72) Inventor: Stroebech, Esben, 2970, Hoersholm (DK); Bach, Anders, 2300, Copenhagen S (DK); Lykke, Mads, 2700, Broenshoej (DK); Toftkaer, Astrid, 2860, Soeborg (DK); Buus, Hasse, 3050, Humlebaek (DK); Kongebo, Tom, 3050, Humlebaek (DK)

(57) **Abstract**

A body waste collecting device comprising a collecting pouch and an adhesive wafer for attachment to the body, said adhesive wafer comprising a backing layer and a first adhesive layer, said first adhesive layer comprising a liquid impermeable, moisture permeable adhesive composition wherein the adhesive wafer comprising an additional second adhesive for sealing around a body opening.

## Description

### Field of the Invention

The present invention relates to a collecting device for attachment to human skin and for collecting bodily waste e.g. an ostomy device.

### Background

Collecting devices for collecting bodily waste, ostomy appliances, wound or fistulae drainage bandages or devices for collecting urine are usually in the form of a receptacle, e.g. a bag, pouch or tube for receiving the waste, connected to an adhesive wafer that can be attached to the skin of the patient. The wafer is typically in the form of a backing layer coated on the skin-facing surface with an adhesive layer and the wafer may further be provided with an aperture for accommodating the body opening. The size and shape of said aperture can often be adapted individually to fit the anatomy of the patient.

One of the crucial parts of such devices is the adhesive wafer. The wafer should be able to fit leak proof around the body opening, have good adherence to the skin without unintended detachment from the skin, but at the same time be easy to remove again without damaging the skin. Furthermore, the wafer should be able to follow the movements of the body and be comfortable to wear.

When designing a skin adhesive one of the major issues is to keep the skin relatively dry underneath the adhesive to prevent maceration. Maceration occurs when the skin is unable to get rid of moisture from transpiration and outlet from a body opening. This may result in degradation of the skins barrier function as well as bad adhesion of the device to the skin.

Usually, skin adhesive keeps the skin dry by absorbing moisture. Absorbing particles or hydrocolloids (HC) are mixed into an adhesive matrix in order to absorb moisture from the skin and thereby the skin is kept relatively dry. This technique is well known in the art and forms the basis for most ostomy adhesives that are commercially available see, e.g. US 4,192,785.

The major problem in using an absorbing adhesive is the changes in the bonding properties when the adhesive absorbs moisture. An absorbing adhesive normally starts out being a well-bonded adhesive and usually ends up being a weak-bonded adhesive after absorbing moisture. This effect is particularly a problem for hydrocolloid-based adhesives. As the adhesive absorbs moisture from the skin, the hydrocolloids swell and take up an increasing amount of space in the skin-bonding zone, thereby reducing the skin bonding effect of the adhesive.

The weak-bonded adhesive will usually still keep the collecting device attached to the skin, however the risk of leakage of bodily waste along the skin surface will increase drastically.

An alternative to the absorbing adhesives described above is a liquid impermeable, moisture permeable adhesive such as polyurethane, silicone, and polyacrylate.

A liquid impermeable, moisture permeable adhesive does not absorb the moisture but rather permeate the water away from the skin surface. Thus, the swelling effect caused by the hydrocolloids will usually not occur.

When using a liquid impermeable, moisture permeable adhesive one can formulate it to be more or less plastic. If the liquid impermeable, moisture permeable adhesive is crosslinked the ability for flow is limited and adhesion is obtained by wetting and affinity to the skin. In this case the cavities in between the mantles and other irregularities will not be filled out by the adhesive by simple flow. Thus, a less resistant adhesive to retain the output and avoid undermining the adhesive with output is seen.

Hence, there is a need to find a solution to avoid leakage of bodily waste occurred because of weak-bonded adhesive and still maintain the ability to use conventional waste collecting devices to adhere to the skin.

It has now surprisingly been found that the collecting device according to the invention provides a sealing effect that solves this problem.

### Summary of the invention

The present invention relates to a body waste collecting device comprising a collecting pouch and an adhesive wafer for attachment to the body, said adhesive wafer comprising a backing layer and a first adhesive layer, said first adhesive layer comprising a liquid impermeable, moisture permeable adhesive composition wherein the adhesive wafer comprising an additional second adhesive for sealing around a body opening.

### Brief description of the drawing

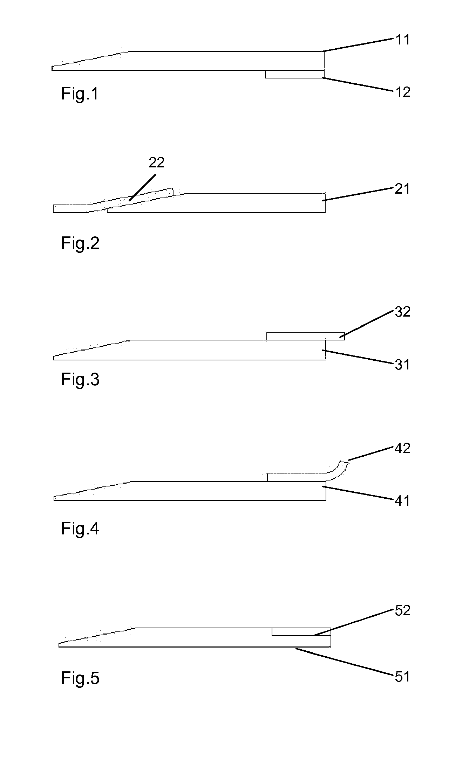

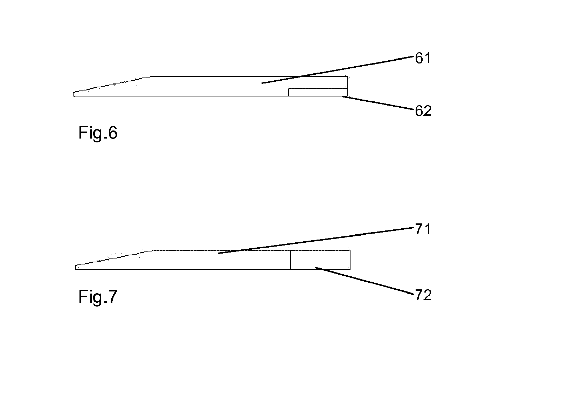

The invention is disclosed more in detail with reference to the drawings in which Figures 1-7 show different embodiments of the invention.

### Detailed description of the present invention

The aim of the invention is to provide a body waste collecting device that includes an additional second adhesive as sealing element.

By body waste collecting device is meant a device being able to collect and hold the output in a collecting item for a predefined time. The holding in place of the device may be obtained by a skin adhesive and the collection may be obtained by a bag.

In an embodiment of the invention a body waste collecting device comprising a collecting pouch and an adhesive wafer for attachment to the body, said adhesive wafer comprising a backing layer and a first adhesive layer, said first adhesive layer comprising a liquid impermeable, moisture permeable adhesive composition wherein the adhesive wafer comprising an additional second adhesive for sealing around a body opening.

The additional second adhesive is used for security and protection for the user in order to avoid leakages and to obtain longer wear time.

The collecting device according to the invention combines the mechanical and adhesive properties of the two kinds of known skin adhesives as described above. The negative effect of the swelling of the hydrocolloid-based skin adhesive is used according to the invention in a beneficial way to provide a sealing effect for the first adhesive layer of a liquid impermeable, moisture permeable adhesive.

In the use of an ostomy appliance the requirements from the adhesive in the peri stomal area are often different as the use varies within stoma type, body shape, user patterns etc.

When an overall soft and pliable adhesive is wanted, a liquid impermeable, moisture permeable adhesive composition as the first adhesive layer can be chosen.

According to the invention a sealing effect through swelling is achieved in order to minimize the exposure of output to the peri stomal area. For example by the use of a hydrocolloid adhesive as the additional second adhesive according to the invention.

Other solutions according to the invention can be a hydrocolloid, a super absorber or a hydrogel based adhesive. When using such a solution the peri stomal area of the adhesive wafer tends to be stiffer resulting in a fixation of the skin in the inner part of the wafer and a more soft and flexible part in the outer zone. The slightly stiffer inner zone is typically obtained from the high filler rate of absorbing particles and choice of adhesive materials that are stiffer than the liquid impermeable, moisture permeable adhesive composition. The collecting device according to the invention substantially maintains the softness given by the liquid impermeable, moisture permeable adhesive composition.

Such an embodiment according to the invention is especially beneficial when used in cases where very liquid or skin aggressive output is exposed to the inner hole of the adhesive wafer for a long time. A typical example is the use of a 2-piece appliance for ileo ostomy where the adhesive wafer will stay on the skin for several days.

By permeable adhesive is meant an adhesive where the moisture vapour transmission rate, MVTR is higher than 100g/m²/24h as measured using the method disclosed herein.

The adhesive used in the first adhesive layer of the present invention has a high moisture vapour transmission rate, preferably a MVTR over 100 g/m²/24hrs, which makes it breathable and very skin friendly. The high moisture transmission of the adhesive is a particular advantage where a medical device has to be worn on the skin for a long time, e.g. days.

According to an embodiment of the invention the first adhesive layer comprises a liquid impermeable, moisture permeable adhesive composition, which comprises a moisture permeable polymer selected from the group of but not limited to polypropyleneoxide, polyurethane, silicone, polyacrylate, ethylene vinyl acetate, and mixtures thereof.

As used herein a moisture permeable polymer means a polymer that absorbs less than 5% in wt, preferably less than 1%, at equilibrium and has a moisture vapour transmission rate of greater than 100 g/m²/24hrs, preferably greater than 200 g/m²/24hrs.

According to an embodiment of the invention the water vapour permeability of the first adhesive layer comprising the liquid impermeable, moisture permeable adhesive composition is higher than 100g/m²/24h as defined herein.

As used herein liquid impermeable means that water is rejected from penetrating the substance by simple flow.

In one embodiment of the invention, the moisture permeable polymer is crosslinked.

As used herein a crosslink means a small region in a macromolecule (polymer chain structure) from which more than 2 chains emanate. The linking may be covalent, physical or ionic.

In another embodiment of the invention, the liquid impermeable, moisture permeable adhesive composition comprises a block copolymer.

As used herein a block copolymer means a copolymer in which the repeating units in the main chain occur in blocks, e.g., -(a)m-(b)n-(a)p-(b)q- , where a and b represent the repeating units and m, n, p, q, are numbers.

In a preferred embodiment of the invention, the liquid impermeable, moisture permeable adhesive composition comprises polypropyleneoxide.

In a preferred embodiment of the invention, the liquid impermeable, moisture permeable adhesive composition comprises polyurethane.

In a preferred embodiment of the invention, the liquid impermeable, moisture permeable adhesive composition comprises ethylene vinyl acetate.

The adhesive composition comprising ethylene vinyl acetate may suitably be an adhesive known in the art such as the adhesive composition disclosed, for example in Danish patent application PA 2007 01003.

In a preferred embodiment of the invention, the liquid impermeable, moisture permeable adhesive composition comprises silicone.

In a preferred embodiment of the invention, the liquid impermeable, moisture permeable adhesive composition comprises polyacrylate.

According to an embodiment of the invention, the additional second adhesive is a paste, a hydrocolloid pressure sensitive adhesive or a liquid impermeable, moisture permeable adhesive composition.

A standard and conventional hydrocolloid pressure sensitive adhesive is a skin friendly adhesive that is capable of adhering to the skin, handling perspiration by absorbing the moist and being removable from the skin without essential damage.

A typical pressure sensitive adhesive is based on a polyisobutylene, PIB (adhering substance) and a carboxy methyl cellulose, CMC (absorbing media).

A typical pressure sensitive adhesive as used today is a highly filled hydrocolloid system with a matrix of polymers that exhibit flow as well as cohesive properties. The hydrocolloids will absorb the moisture from perspiration and output from the body and the polymers will adhere to the skin through skin polymer affinity and the polymers will flow into the small cavities of the skin. The cohesion of the adhesive will ensure that erosion is minimized and that the wafer will be removed in one piece and that minimal adhesive residues are left at the skin.

As used herein a paste is a moldable or flowable adhesive that is able to adhere to the skin, flow or being permanently pressed into irregularities of the skin and thus protecting the skin from the output. A paste is usually used as an accessory to stoma care products for sealing around the stoma.

According to another embodiment of the invention, the additional second adhesive is a liquid impermeable, moisture permeable adhesive composition comprising a moisture permeable polymer selected from the group of polypropyleneoxide, polyurethane, silicone, polyacrylate, ethylene vinyl acetate, and mixtures thereof.

According to one embodiment of the invention, the additional second adhesive is another liquid impermeable, moisture permeable adhesive composition compared to the composition of the first adhesive layer.

It may be advantageous that the additional second adhesive comprises absorbent particles. According to an embodiment of the invention the additional second adhesive comprises absorbent particles.

The particles may be absorbent articles such as hydrocolloids, microcolloids or super absorbers in order for the composition to absorb moisture from skin.

Microcolloid particles are well-known in the art e.g. from International Patent Application No. WO 02/066087, which discloses adhesive compositions comprising microcolloid particles. The microcolloid particles may have a particle size of less than 20 microns.

The additional second adhesive may comprise 1-60 % w/w of hydrocolloid (HC) or super absorbent particles (SAP) particles, more preferred 30-60% w/w particles.

According to a preferred embodiment of the invention, the additional second adhesive comprises absorbent particles.

According to a preferred embodiment of the invention, the absorbent particles are selected from hydrocolloids, microcolloids and super absorbers.

According to a preferred embodiment of the invention, the additional second adhesive is a hydrocolloid pressure sensitive adhesive.

In an embodiment of the invention, the additional second adhesive is located on the skin-facing surface of the first adhesive layer.

By the skin-facing surface of the adhesive is meant the side adhering to the skin.

By located is meant where the additional second adhesive is placed on the collecting device and specifies the position of the additional second adhesive on the collecting device.

In another embodiment of the invention, the additional second adhesive is located on the pouch-facing surface of the adhesive wafer.

By the pouch-facing surface or non-skin-facing surface is meant the side of the adhesive or backing pointing away from the skin (non-bonding side).

According to another embodiment of the invention, the additional second adhesive is located at the inner rim of the adhesive wafer.

By the inner rim portion of the adhesive wafer is meant the portion in the perianal, peristomal or fistal area. This area of the adhesive is exposed to faeces as well as perspiration. The properties of the inner rim portion depend on wear time, output type and anatomy among others.

According to one embodiment of the invention, the additional second adhesive is located at the outer rim of the adhesive wafer.

By the outer rim portion of the adhesive wafer is meant the portion essentially outside the welding zone or coupling. This portion is less exposed to faeces but has to handle perspiration as well as mechanical exposure due to movements and pull from the bag.

According to an embodiment of the invention, the additional second adhesive is in the form of a ring.

By an adhesive ring is meant an adhesive, essentially surrounding the body opening that needs to be drained or a ring covering the outer or inner rim of the adhesive wafer

According to an embodiment of the invention, a hole of the ring has a diameter being less than the diameter of a hole in the adhesive wafer with the first adhesive layer. By this embodiment the additional second adhesive will have a tighter fit around the body opening compared to the adhesive wafer and compared to the first adhesive layer. In one embodiment the sealing and the tighter fit of the additional second adhesive is obtained by turtle necking.

According to one embodiment of the invention, the additional second adhesive is part of a ring such as a half ring covering 180 degrees of a circle.

According to an embodiment of the invention, the additional second adhesive covers the entire skin-facing surface of the first adhesive layer.

Optionally, the additional second adhesive only covers a part of the first adhesive layer.

According to an embodiment of the invention, the additional second adhesive partly covers the adhesive wafer.

According to another embodiment of the invention, the additional second adhesive partly covers the skin-facing surface of the first adhesive layer.

According to another embodiment of the invention the adhesive wafer has a recess for adapting the additional second adhesive.

The collecting device could be pre-shaped with a recess that fits the additional second adhesive to be built in the device. A recess could be a cut-out of part of the first adhesive layer.

By adapting is meant that the additional second adhesive is cast within the collecting device.

According to an embodiment of the invention, the device has an optional backing layer for the additional second adhesive.

According to another embodiment of the invention, the additional second adhesive is integrated in the wafer.

Sealing an opening refers to protection of an opening from output, for example use of a paste around a stoma at the rim of the perinea skin or the rim of a fistula.

According to an embodiment of the invention, the body opening is a stoma.

According to another embodiment of the invention, the body opening is a fistula.

According to another embodiment of the invention, the body opening is an anus.

By a body opening is meant a natural or artificial body opening such as an anus, a stoma or a fistula.

According to an embodiment of the invention, the collecting device is an ostomy appliance.

According to another embodiment of the invention, the collecting device is a faecal collecting device.

According to another embodiment of the invention, the collecting device is a fistula collecting device.

According to an embodiment of the invention, the collecting pouch is detachable.

According to another embodiment of the invention, the collecting pouch is integrated with the wafer.

The collecting pouch may be detachable from the adhesive wafer by a coupling system or the pouch and the wafer may be integrated with the wafer, e.g. by welding. The two versions are known as one piece or two-piece appliances for ostomy.

### Description of the preferred embodiments

The invention is now explained more in detail with reference to the drawings showing preferred embodiments of the invention.
Figures 1-7 are all drawings of a side view of the left half of the adhesive wafer according to the invention. All wafers are covered with a backing layer. The skin-facing surface is illustrated in the drawings as the downward side of the wafer and the backing layer is placed on top of the wafer.
Figure 1 shows an adhesive wafer with a first adhesive layer (11) and an additional second adhesive (12) where the additional second adhesive only covers part of the adhesive wafer and is placed on the surface of the skin-facing adhesive wafer. The additional second adhesive can optionally be covered by a second backing layer placed in between the non-skin-facing side of the additional second adhesive and the skin-facing side of the first adhesive layer.
Figure 2 illustrates a side view of an adhesive wafer with a first adhesive layer (21) partly covered with an additional second adhesive (22) in the outer rim portion of the wafer.
Figure 3 illustrates a side view of an embodiment of the invention, where the hole of the ring of the additional second adhesive (32) has a diameter being less than the hole in the adhesive wafer with the first adhesive layer (31).
Figure 4 illustrates a side view of another embodiment of the invention, where the hole of the ring of the additional second adhesive (42) has a diameter being less than the hole in the adhesive wafer with the first adhesive layer (41).
Figure 5 illustrates a side view of an adhesive wafer with a first adhesive layer (51) having a recess for adapting the additional second adhesive (52).
Figure 6 illustrates a side view of another embodiment of the invention where an adhesive wafer with a first adhesive layer (61) has a recess for adapting the additional second adhesive (62).
Figure 7 illustrates a side view of an embodiment of the invention where an adhesive wafer with a first adhesive layer (71) is in combination with an additional second adhesive (72) in the inner portion of the adhesive wafer.

### Methods

### Determination of water absorption

In order to get better correlation between measured water absorption and actual performance in a humanlike environment, a modified version of the ISO 62 standard was used: Pieces of adhesive of 1x25x25 mm³ were fastened on a piece of glass using double sided adhesive and the constructs were immersed in saline water (0.9% NaCI in dematerialized water) at 32°C. After 24 hours the samples were removed and carefully dripped dry and weighed. The change in weight was recorded and reported as weight gain in percent of the original dry weight of the adhesive. In the following we will call this value W₂₄ₕ

### Determination of moisture vapour transmission rate (MVTR)

MVTR was measured in grams per square meter (g/m²) over a 24 hours period using an inverted Paddington cup method (British Pharmacopoeia, 1993,Addendum 1996, page 1943. HMSO London): A container or cup being water and water vapour impermeable having an opening was used. 20ml saline water (0.9%NaCI in demineralised water) was placed in the container and the opening was sealed with the test adhesive film. The container, with a duplicate, was placed into an electrically heated humidity cabinet and the container or cup was placed up side down in a way that the water was in contact with the adhesive. The cabinet was maintained at 37°C and 15% relative humidity (RH). After about an hour, the containers were considered to be in equilibrium with the surroundings and were weighed. 24h after the first weighing, the containers were weighed again. The difference in weight was due to evaporation of vapour transmitted through the adhesive film. This difference was used to calculate Moisture vapour transmission rate or MVTR. MVTR was calculated as the weight loss after 24h divided by the area of the opening in the cup (g/m²/24h). If the adhesive film could not support the weight of the water, a supporting film with very high permeability was used as support.

### Example 1

A standard hydrocolloid adhesive as disclosed in International Patent Application No. WO 2007/082538 or a paste as disclosed in International Patent Application No. WO 98/17329, in the dimension of a round oblate with a diameter of 50 mm and a thickness of 1 mm was placed in the central portion of a liquid impermeable, moisture permeable adhesive that was connected to a collecting bag. The device was used for collecting stoma ileo effluent for 24 hours on a stoma-operated person.

In this case the collecting stoma device was perceived as a very soft, flexible, reliable and comfortable device with the sealing effect of the hydrocolloid adhesive in the central portion. No skin conditions were seen and swelling of the hydrocolloid controlled the faeces in a way that it did not undermine the adhesive wafer. Undermining was also prevented by the flowing of the hydrocolloid adhesive into the micropores of the skin.

### Example 2

An acryllic adhesive, 3M1504, in the dimension of a round oblate with a diameter of 50 mm and a thickness of 0,4 mm was placed in the central portion of a liquid impermeable, moisture permeable adhesive that was connected to a collecting bag. The relatively thin acrylic adhesive was permeable to moisture and would transport water by permeability to the liquid impermeable, moisture permeable adhesive. The device was used for collecting stoma ileo effluent for 24 hours on a stoma-operated person.

In this case the collecting stoma device was also perceived as a very soft, flexible, reliable and comfortable device with the sealing effect of the acrylic adhesive in the central portion. Sealing was obtained by the flowing of the acrylic adhesive into the micropores of the skin and moisture from perspiration was transported by diffusion into the liquid impermeable, moisture permeable adhesive. No skin conditions were seen. In one case the hole of the device was cut in a slightly (app 5 mm) lower diameter of the receiving stoma in order to obtain a turtleneck effect as a sealing. This experiment resulted in a good sealing preventing the stoma to enter behind the adhesive wafer, thus irritating the peristomal skin.

### Example 3

A liquid impermeable, moisture permeable adhesive with plastic properties as disclosed in Danish patent application PA 2007 01003, in the dimension of a round oblate with a diameter of 50 mm and a thickness of 1 mm was placed in the central portion of a liquid impermeable, moisture permeable non-flowing adhesive that was connected to a collecting bag. The device was used for collecting stoma ileo effluent for 24 hours on a stoma-operated person.

In this case the collecting stoma device was perceived as a very soft, flexible and comfortable device with the sealing effect of the plastic adhesive in the central portion. No skin conditions were seen. Undermining was prevented by the flow of the plastic adhesive into the skin surface and skin health was obtained by the two liquid impermeable, moisture permeable adhesives.

### Embodiments

1. A body waste collecting device comprising
- a collecting pouch
- an adhesive wafer for attachment to the body, comprising
- a backing layer
- a first adhesive layer,
said first adhesive layer comprising a liquid impermeable, moisture permeable adhesive composition, wherein the adhesive wafer comprising an additional second adhesive for sealing around a body opening.

2. The collecting device according to embodiment 1, wherein the first adhesive layer comprises the liquid impermeable, moisture permeable adhesive composition, which comprises a moisture permeable polymer selected from the group of polypropyleneoxide, polyurethane, silicone, polyacrylate, ethylene vinyl acetate, and mixtures thereof.

3. The collecting device according to any of the preceding embodiments,
wherein the water vapour permeability of the first adhesive layer comprising the liquid impermeable, moisture permeable adhesive composition is higher than 100g/m²/24h as defined herein.

4. The collecting device according to any of embodiments 1-3, wherein the additional second adhesive is a paste, a hydrocolloid pressure sensitive adhesive or a liquid impermeable, moisture permeable adhesive composition.

5. The collecting device according to embodiment 4, wherein the additional second adhesive is a liquid impermeable, moisture permeable adhesive composition comprising a moisture permeable polymer selected from the group of polypropyleneoxide, polyurethane, silicone, polyacrylate, ethylene vinyl acetate, and mixtures thereof.

6. The collecting device according to embodiment 5, wherein the additional second adhesive is another liquid impermeable, moisture permeable adhesive composition compared to the composition of the first adhesive layer.

7. The collecting device according to any of embodiments 4-6, wherein the additional second adhesive comprises absorbent particles.

8. The collecting device according to embodiment 7, wherein the absorbent particles are selected from hydrocolloids, microcolloids and super absorbers.

9. The collecting device according to embodiment 4, wherein the additional second adhesive is a hydrocolloid pressure sensitive adhesive.

10. The collecting device according to any of embodiments 1-9, wherein the additional second adhesive is located on the skin-facing surface of the first adhesive layer.

11. The collecting device according to any of embodiments 1-9, wherein the additional second adhesive is located on the pouch-facing surface of the adhesive wafer.

12. The collecting device according to any of embodiments 1-11, wherein the additional second adhesive is located at the outer rim of the adhesive wafer.

13. The collecting device according to any of embodiments 1-11, wherein the additional second adhesive is located at the inner rim of the adhesive wafer.

14. The collecting device according to any of embodiments 1-13, wherein the additional second adhesive is in the form of a ring.

15. The collecting device according to embodiment 14, wherein a hole of the ring has a diameter being less than the diameter of a hole in the adhesive wafer.

16. The collecting device according to any of embodiments 1-15, wherein the additional second adhesive fully covers the first adhesive layer on the skin-facing surface.

17. The collecting device according to any of embodiments 1-15, wherein the additional second adhesive partly covers the first adhesive layer on the skin-facing surface.

18. The collecting device according to any of embodiments 1-15, wherein the additional second adhesive partly covers the adhesive wafer.

19. The collecting device according to any of embodiments 1-18, wherein the adhesive wafer has a recess for adapting the additional second adhesive.

20. The collecting device according to any of embodiments 1-19, wherein the device has an optional backing layer for the additional second adhesive.

21. The collecting device according to any of embodiments 1-20, wherein the additional second adhesive is integrated in the wafer.

22. The collecting device according to any of embodiments 1-21, wherein the body opening is a stoma.

23. The collecting device according to any of embodiments 1-21, wherein the body opening is a fistula.

24. The collecting device according to any of embodiments 1-21, wherein the body opening is an anus.

25. The collecting device according to any of embodiments 1-22, wherein the collecting device is an ostomy appliance.

26. The collecting device according to any of embodiments 1-22, 24, wherein the collecting device is a faecal collecting device.

27. The collecting device according to any of embodiments 1-21, 23, wherein the collecting device is a fistula collecting device.

28. The collecting device according to any of embodiments 1-27, wherein the collecting pouch is detachable.

29. The collecting device according to any of embodiments 1-27, wherein the collecting pouch is integrated with the wafer.

## Claims

1. A body waste collecting device comprising
- a collecting pouch
- an adhesive wafer for attachment to the body, comprising
- a backing layer
- a first adhesive layer (11,21,31,41,51,61 respectively 71),
said first adhesive layer comprising a liquid impermeable, moisture permeable adhesive composition (12,22,32,42,52,62 respectively 72), **characterized in that** the adhesive wafer comprising an additional second adhesive for sealing around a body opening, said additional second adhesive is a paste

2. The collecting device according to claim 1, wherein the first adhesive layer (11,21,31,41,51,61 respectively 71) comprises the liquid impermeable, moisture permeable adhesive composition, which comprises a moisture permeable polymer selected from the group of polypropyleneoxide, polyurethane, silicone, polyacrylate, ethylene vinyl acetate, and mixtures thereof.

3. The collecting device according to any of the preceding claims, wherein the water vapour permeability of the first adhesive layer (11,21,31,41,51,61 respectively 71) comprising the liquid impermeable, moisture permeable adhesive composition is higher than 100g/m²/24h as defined herein.

4. The collecting device according to any of claims 1-3, wherein the additional second adhesive (12,22,32,42,52,62 respectively 72) comprises absorbent particles.

5. The collecting device according to claim 4, wherein the absorbent particles are selected from hydrocolloids, microcolloids and super absorbers.

6. The collecting device according to any of claims 1-5, wherein the additional second adhesive (12 respectively 62) is located on the skin-facing surface of the first adhesive layer (11 respectively 61).

7. The collecting device according to any of claims 1-5, wherein the additional second adhesive (22,32,42,52 respectively 72) is located on the pouch-facing surface of the adhesive wafer.

8. The collecting device according to any of claims 1-7, wherein the additional second adhesive (22) is located at the outer rim of the adhesive wafer.

9. The collecting device according to any of claims 1-7, wherein the additional second adhesive (12,32,42,52,62 respectively 72) is located at the inner rim of the adhesive wafer.

10. The collecting device according to any of claims 1-9, wherein the additional second adhesive (12,22,32,42,52,62 respectively 72) is in the form of a ring.

11. The collecting device according to claim 10, wherein a hole of the ring has a diameter being less than the diameter of a hole in the adhesive wafer.

12. The collecting device according to any of claims 1-11, wherein the additional second adhesive fully covers the first adhesive layer on the skin-facing surface.

13. The collecting device according to any of claims 1-11, wherein the additional second adhesive (12 respectively 62) partly covers the first adhesive layer (11 respectively 61) on the skin-facing surface.

14. The collecting device according to any of claims 1-11, wherein the additional second adhesive (12,22,32,42,52,62 respectively 72) partly covers the adhesive wafer.

15. The collecting device according to any of claims 1-14, wherein the adhesive wafer has a recess for adapting the additional second adhesive (52 respectively 62).

16. The collecting device according to any of claims 1-15, wherein the device has an optional backing layer for the additional second adhesive (12,22,32,42,52,62 respectively 72).

17. The collecting device according to any of claims 1-16, wherein the additional second adhesive (12,22,32,42,52,62 respectively 72) is integrated in the wafer.

18. The collecting device according to any of claims 1-17, wherein the body opening is a stoma.

19. The collecting device according to any of claims 1-17, wherein the body opening is a fistula.

20. The collecting device according to any of claims 1-17, wherein the body opening is an anus.

21. The collecting device according to any of claims 1-18, wherein the collecting device is an ostomy appliance.

22. The collecting device according to any of claims 1-18, 20, wherein the collecting device is a faecal collecting device.

23. The collecting device according to any of claims 1-17, 19, wherein the collecting device is a fistula collecting device.

24. The collecting device according to any of claims 1-23, wherein the collecting pouch is detachable.

25. The collecting device according to any of claims 1-23, wherein the collecting pouch is integrated with the wafer.
